# EUROPÄISCHE PATENTSCHRIFT

(11) **EP 2 241 243 B1**
(45) Veröffentlichungstag und Bekanntmachung des Hinweises auf die Patenterteilung: **04.04.2012**
(21) Anmeldenummer: 10153309.9
(22) Anmeldetag: 11.02.2010
(51) Int. Cl.: A61B 1/002, A61B 1/12

(54) **Endoskop mit elektrischem Heizsystem**
Endoscope with electrical heating system
Endoscope doté d'un système de chauffage électrique

(30) Priorität: 16.04.2009 DE 102009017606
(43) Veröffentlichungstag der Anmeldung: 20.10.2010
(73) Patentinhaber: Henke-Sass, Wolf GmbH, 78532 Tuttlingen (DE)
(72) Erfinder: Seeh, Daniel, 78194, Immendingen (DE); Pfaff, Armin, 78655, Seedorf (DE); Häckl, Norbert, 88637, Leibertingen (DE); Weller, Thomas, 78532, Tuttlingen (DE)
(74) Vertreter: Geyer, Werner

(56) Entgegenhaltungen:
- EP-A2- 1 803 388
- JP-A- 2002 291 684
- JP-A- 2006 000 282
- US-A- 4 076 018
- US-A- 4 722 000
- US-B1- 6 712 479
- "Flexible tube used as endoscope guide tube etc. - comprises porous PTFE inner layer, air-tight layer of fluoro rubber elastomer, epichlorohydrin elastomer and conductive layer of metal" DERWENT, 26. Januar 1987 (1987-01-26), XP002303694

## Beschreibung

Endoskope weisen in der Regel ein Rohr auf, in dem eine Abbildungsoptik angeordnet ist. Um die Abbildungsoptik zu schützen, ist das distale Ende des Rohres häufig mit einem Deckglas verschlossen.

Im Betrieb kann das Deckglas beschlagen, wenn z.B. das Endoskop, das Zimmertemperatur aufweist, in eine wärmere Körperhöhle eingeführt wird.

Um ein solches Beschlagen des Deckglases zu verhindern, ist es bekannt, ein elektrisches Heizsystem zum Erwärmen des Deckglases vorzusehen. Aus der US 4,076,018 ist beispielsweise ein Endoskop mit den Merkmalen des Oberbegriffes des Anspruches 1 bekannt. Jedoch ist es notwendig, elektrische Leitungen durch das Endoskoprohr zu führen, um die auf der Innenseite des Deckglases aufgebrachte elektrische leitfähige Beschichtung mit Strom beaufschlagen zu können. Diese notwendige Verkabelung ist aufwendig und führt daher zu höheren Herstellungskosten.

Die EP 1 803 388 A2 beschreibt ein Endoskop mit einem Heizelement im distalen Endbereich des Endoskoprohres. Auch die JP 2002-291684 sowie die JP 2006-282 beschreiben jeweils Endoskope mit einem Heizelement im distalen Endbereich des Endoskopschaftes.

Die US 6,712,479 B1 beschreibt eine elektrische Heizvorrichtung im Bereich der proximalen Linse eines Laparoskopes und die US 4,722,000 beschreibt ein elektrisches Heizsystem in einem Adapter für eine Endoskopkamera. Die JP 62-017486 beschreibt ein flexibles Rohr für Endoskope, das eine poröse PTFE-Innenschicht, eine luftdichte Schicht aus einem Fluorkautschuk-Elastomer und einem Epichlorhydrin-Elastomer sowie eine leitende Schicht aus Metall aufweist.

Ausgehend hiervon ist es Aufgabe der Erfindung, ein Endoskop der eingangs genannten Art so weiter zu bilden, daß es kostengünstig hergestellt und bei dem das Beschlagen des Deckglases sicher verhindert werden kann.

Erfindungsgemäß wird die Aufgabe bei einem Endoskop der eingangs genannten Art durch die Merkmale des kennzeichnenden Teils des Anspruches 1 gelöst.

Aufgrund der Kontaktierung des ersten Kontaktabschnittes über die elektrisch leitfähige Fassung ist eine leicht herstellbare und äußerst platzsparende Kontaktierung realisierbar. Ferner ist keine zusätzliche Verkabelung zur Kontaktierung des ersten Kontaktabschnittes notwendig, da das erste Rohr elektrisch leitfähig ist und die Fassung mit dem dem ersten Kontaktabschnitt zugeordneten Anschluß elektrisch verbindet. Ferner trägt auch das elektrisch leitfähige zweite Rohr, das die elektrische Verbindung des zweiten Kontaktabschnittes mit dem zugeordneten Anschluß verwirklicht, dazu bei, die Kontaktierung der leitfähigen Beschichtung äußerst platzsparend zu realisieren. Es werden nämlich für die elektrische Kontaktierung die für das Endoskop notwenigen Rohre eingesetzt.

Mit dem erfindungsgemäßen Heizsystem kann das Deckglas leicht auf die gewünschte Temperatur gebracht und gehalten werden. Die elektrisch leitfähige Beschichtung dient als Widerstandsheizung, mit der z. B. eine Deckglas-Temperatur im Bereich von 37° - 42°C erzeugt werden kann.

Der erste Kontaktabschnitt steht bevorzugt im direkten Kontakt mit der Fassung, um die elektrische Verbindung des ersten Kontaktabschnittes mit der Fassung herzustellen.

Die Fassung kann als separates Bauelement ausgebildet sein, das mit dem ersten Rohr verbunden ist. Es ist jedoch auch möglich, die Fassung einstückig mit dem ersten Rohr auszubilden, so daß das distale Ende des ersten Rohres selbst die Fassung bildet.

Die beiden Rohre können insbesondere als Edelstahlrohre ausgebildet sein.

Es kann eine Feder (beispielsweise Spiralfeder) zwischen dem zweiten Kontaktabschnitt und dem zweiten Rohr angeordnet sein, die eine elektrische Verbindung des zweiten Kontaktabschnittes mit dem zweiten Rohr bewirkt. Durch diese Art der Kontaktierungen können beispielsweise unterschiedliche thermische Ausdehnungen des ersten und zweiten Rohres kompensiert werden.

Bei dem erfindungsgemäßen Endoskop kann das erste Rohr hermetisch dicht verschlossen sein. Darunter wird hier insbesondere verstanden, daß das erste Rohr autoklavierbar ist. Beim Autoklavieren wird das Endoskop bzw. das erste Rohr mindestens mehrere Minuten gesättigtem Wasserdampf von circa 120°- 140°C zur Sterilisation ausgesetzt, ohne daß dabei das Endoskop beschädigt wird (ohne daß insbesondere Wasserdampf in das Innere des ersten Rohres eintreten kann). In diesem Fall ist das Endoskop bei medizinischen Anwendungen sehr kostengünstig, da es durch das Autoklavieren sehr schnell optimal sterilisiert und somit häufig eingesetzt werden kann.

Die Beschichtung kann, in Draufsicht auf das Deckglas gesehen, die Form eines offenen Ringes aufweisen. Damit kann die Erstreckung der Beschichtung möglichst groß gewählt werden, um die gewünschte Widerstandsheizung optimal realisieren zu können.

Insbesondere kann bei dem Endoskop im ersten Rohr am distalen Ende eine Abbildungsoptik angeordnet sein, die so ausgelegt ist, daß zur Abbildung eines vor dem Deckglas befindlichen Objektes nur Licht verwendet wird, das einen vorbestimmten Teilbereich der Innenseite durchläuft, wobei die Beschichtung außerhalb des Teilbereichs auf der Innenseite aufgebracht ist. Dadurch ist die Beschichtung nicht in dem von der Abbildungsoptik optisch genutzten Bereich (= vorbestimmter Teilbereich) der Innenseite des Deckglases angeordnet, so daß die Beschichtung keine unerwünschten Abschattungen erzeugt.

Das Deckglas ist insbesondere ein Saphirglas.

Das Endoskop kann als starres oder flexibles Endoskop ausgebildet sein. So kann das Endoskop bzw. das erste Rohr abwinkelbar ausgebildet sein. Insbesondere kann das distale Ende des ersten Rohres abwinkelbar sein.

Das Endoskop kann einen flächigen Bildsensor aufweisen, der der Abbildungsoptik nachgeordnet ist. Der Bildsensor kann beispielsweise im distalen Ende des ersten Rohres angeordnet sein. Er kann aber auch an jeder anderen geeigneten Position im Endoskop vorgesehen sein.

Alternativ ist es möglich, daß zwischen der Abbildungsoptik und dem proximalen Ende des Endoskops eine Übertragungsoptik (z.B. Relais-Optik) vorgesehen ist, die dafür sorgt, daß das mittels der Abbildungsoptik erzeugte Bild des Objektes bis zum proximalen Ende übertragen wird und dort für einen Benutzer beispielsweise erfaßbar ist oder mittels einer am proximalen Ende des Endoskops vorzusehenden Videokamera aufnehmbar ist.

Die Strom- bzw. Spannungsquelle, die mit den zwei elektrischen Anschlüssen des Heizsystems verbindbar ist, kann eine separate Quelle oder im Endoskop integriert sein. Die Spannung, die die Quelle zwischen beide Anschlüsse anlegt, kann im Bereich von 0,8 - 1,5 V liegen (insbesondere z.B. 1,25 V), wobei ein Strom von ca. 0,5 - 0,9 A (insbesondere 0,75 A) fließt.

Die Beschichtung kann als Metallisierung ausgebildet sein. So kann z.B. eine Chromschicht vorgesehen werden. Um eine optimale Haftung der Chromschicht auf einem Saphir-Deckglas zu erzielen, kann zwischen dem Deckglas und der Chromschicht eine Siliziumoxid-Schicht vorgesehen sein. In den Kontaktabschnitten kann anstatt der Chromschicht oder auf der Chromschicht eine Goldbeschichtung ausgebildet sein, um eine optimale Kontaktierung zu gewährleisten.

Abschließend kann noch eine Antireflexbeschichtung (natürlich nicht auf den Kontaktabschnitten) über die gesamte Innenseite vorgesehen sein, die im Bereich der leitfähigen Beschichtung als Schutzschicht und im restlichen Bereich der Innenseite des Deckglases als Antireflexschicht zur Unterdrückung von unerwünschtem Streulicht dient.

Das Heizsystem kann einen Regler aufweisen, mit dem z.B. die durch die elektrische Beschichtung fließende Stromstärke geregelt wird.

Ferner kann das Heizsystem einen Temperatursensor (beispielsweise nahe am Deckglas) aufweisen, mit dem eine Ist-Temperatur erfaßt wird, die bei der Regelung verwendet wird.

Das Endoskop kann weitere, dem Fachmann bekannte Elemente und Bauteile aufweisen, die für den Betrieb des Endoskops notwendig sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Seitenansicht einer ersten Ausführungsform des erfindungsgemäßen Endoskops;
- Fig. 2: eine vergrößerte Schnittansicht des Details A von Fig. 1;
- Fig. 3: die Schnittansicht entlang der Linie B-B in Fig. 2;
- Fig. 4: eine vergrößerte Schnittansicht des Details C von Fig. 1, und
- Fig. 5: eine vergrößerte Ansicht des Details D von Fig. 4.

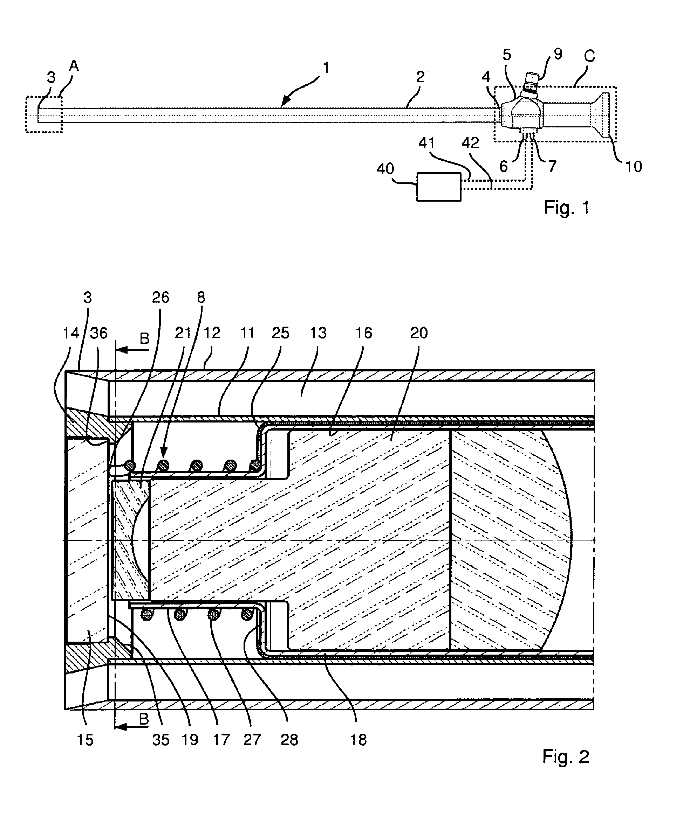

Bei der in Fig. 1 gezeigten Ausführungsform umfaßt das erfindungsgemäße Endoskop 1 einen Schaft 2 mit einem distalen Ende 3 und einem proximalen Ende 4.

Das proximale Ende 4 des Schaftes 2 mündet in einen Handgriff 5, der zwei elektrische Anschlüsse 6, 7 des nachfolgend noch näher beschriebenen Heizsystemes 8, einen Lichtleiteranschluß 9 sowie an dem dem Schaft 2 abgewandten proximalen Ende eine Einblicköffnung 10 aufweist. Die Gesamtlänge des in Fig. 1 gezeigten Endoskops 1 beträgt ca. 200 mm und der Schaftaußendurchmesser beträgt ca. 10 mm.

Wie insbesondere aus der vergrößerten Schnittdarstellung des Details A von Fig. 1 in Fig. 2 ersichtlich ist, umfaßt der Schaft 2 ein erstes Rohr 11, das in einem Außenrohr 12 mit größerem Durchmesser eingesetzt ist, so daß der dadurch vorhandene Zwischenraum 13 zwischen den beiden Rohren 11 und 12 in bekannter Art und Weise zur Aufnahme von Lichtleitern (nicht gezeigt) verwendet werden kann, die zur Beleuchtung des zu betrachtenden Objektes dienen.

Am distalen Ende des ersten Rohres 11 ist eine elektrisch leitfähige Fassung 14 befestigt, in der ein Deckglas 15 eingelötet ist. Die Verbindung zwischen der Fassung 14 und dem ersten Rohr 11 sowie zwischen dem Deckglas 15 und der Fassung 14 ist jeweils so ausgeführt, daß das distale Ende des ersten Rohres 11 hermetisch dicht verschlossen ist.

In dem ersten Rohr 11 ist ein zweites Rohr 16 eingesetzt, das ebenfalls elektrisch leitfähig ist und sich vom Handgriff 5 bis zum distalen Ende 3 hin erstreckt, wobei der Außendurchmesser des zweiten Rohres 16 vom Handgriff 5 bis kurz vor dem distalen Ende 3 nur geringfügig kleiner ist als der Innendurchmesser des ersten Rohres 11. Zwischen beiden Rohren 11, 16 ist eine Isolierschicht 25 ausgebildet, die gewährleistet, daß sich die beiden Rohre 11, 16 nicht berühren, so daß zwischen ihnen kein direkter elektrischer Kontakt vorliegt.

Wie in der Darstellung von Fig. 2 ersichtlich ist, weist das zweite Rohr 16 einen vorderen Abschnitt 17 auf, dessen Außendurchmesser kleiner ist als der Außendurchmessers des restlichen Teils 18 des zweiten Rohres 16. Bei der hier gezeigten Ausführungsform ist der Außendurchmesser des vorderen Abschnittes 17 circa um ein Drittel kleiner als der Außendurchmesser des restlichen Abschnitts 18 des zweiten Rohres 16. Das distale Ende des vorderen Abschnittes 17 ist von der Innenseite 19 des Deckglases 15 beabstandet. Das gleiche trifft auf die im zweiten Rohr 16 angeordnete Optik 20 zu, da das am nähesten am Deckglas 15 liegende Optikelement 21 der Optik 20 von dem Deckglas 15 beabstandet ist. Es liegt somit ein Spalt zwischen dem Deckglas 15 und dem Optikelement 21 vor.

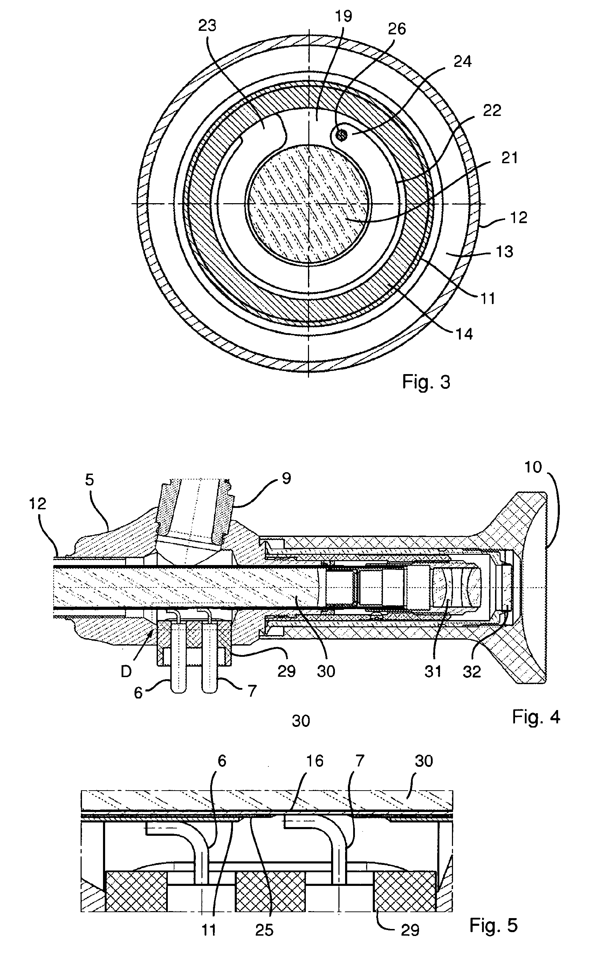

In Fig. 3 ist die Schnittansicht entlang der Schnittlinie B-B von Fig. 2 gezeigt. Dieser Schnittdarstellung in Fig. 3 kann entnommen werden, daß auf der Innenseite 19 des Deckglases 15 eine elektrisch leitfähige Beschichtung bzw. Metallisierung 22 aufgebracht ist. Die Beschichtung erstreckt sich ringförmig auf der Innenseite 19, wobei jedoch die Ringform nicht komplett geschlossen, sondern offen ist. In der Darstellung von Fig. 3 ist gut erkennbar, daß der Innendurchmesser der Beschichtung 22 größer ist als der Außendurchmesser des Optikelementes 21. Dadurch kann sichergestellt werden, daß die Beschichtung 22 zu keiner nachteiligen Abschattung für die Optik 20 bei Abbildung eines Objektes durch das Deckglas 15 hindurch führt.

Die Beschichtung 22 weist einen ersten und zweiten Kontaktabschnitt 23, 24 auf. Der erste Kontaktabschnitt 23 erstreckt sich bis zum Rand des Deckglases 15. Da das Deckglas 15, wie insbesondere in Fig. 2 ersichtlich ist, mit dem äußeren Abschnitt (hier ein ringförmiger Abschnitt) der Innenseite 19 an der durch eine Schulter 35 der Fassung 14 gebildeten (hier ringförmigen) Sitzfläche 36 liegt, ist der erste Kontaktabschnitt 23 elektrisch mit der Fassung 14 verbunden.

Auf dem zweiten Kontaktabschnitt 24 liegt das vordere Ende 26 einer Schraubenfeder 27 an, die sich entlang des vorderen Abschnitts 17 des zweiten Rohres 16 bis zu einem Schulterbereich 28 des dritten Rohres erstreckt. Der Schulterbereich 28 verbindet den vorderen Abschnitt 17 des zweiten Rohres 16 mit dem restlichen Abschnitt 18 des zweiten Rohres 16. Die Schraubenfeder 27 ist aus einem elektrisch leitfähigen Material gebildet und/oder mit einem solchen beschichtet und stellt somit einen elektrischen Kontakt zwischen dem zweiten Kontaktabschnitt 24 und dem zweiten Rohr 16 her. Durch diese Kontaktierung mittels der Feder 27 kann vorteilhaft eine Kontaktierung selbst bei temperaturbedingten unterschiedlichen Ausdehnungen des zweiten und ersten Rohres 16, 11 sichergestellt werden.

Somit liegt ein elektrischer Kontakt zwischen dem ersten Kontaktabschnitt 23 und dem ersten Rohr 11 sowie zwischen dem zweiten Kontaktabschnitt 24 und dem zweiten Rohr 16 vor. Wie bereits erwähnt, sind das erste und zweite Rohr 11, 16 aufgrund der dazwischen vorgesehenen Isolierschicht 25 elektrisch voneinander getrennt.

In Fig. 4 ist das Detail C von Fig. 1 in einer vergrößerten Schnittansicht dargestellt. Dieser Darstellung kann entnommen werden, daß das erste Rohr 11 elektrisch mit dem ersten elektrischen Anschluß 6 verbunden ist. Das zweite Rohr 16 ist elektrisch mit dem zweiten Anschluß 7 verbunden. Dazu ist, wie insbesondere in der vergrößerten Ansicht des Details D in Fig. 5 ersichtlich ist, in dem ersten Rohr 11 sowie in der Isolierschicht 25 eine Unterbrechung ausgebildet, so daß die gewünschte elektrische Kontaktierung zwischen dem zweiten Rohr 16 und dem zweiten elektrischen Anschluß 7 vorliegt.

Die beiden Anschlüsse 6, 7 sind in einem Anschlußblock 29 (Fig. 4) gehalten und von außerhalb des Endoskops 1 zugänglich. Um die gewünschte hermetische Dichtheit des ersten Rohres 11 zu gewährleisten, ist der freigelegte Bereich beispielsweise so mit einem Dichtmaterial (nicht gezeigt) abgedichtet.

Das Heizsystem 8 umfaßt bei der beschriebenen Ausführungsform die beiden Anschlüsse 6, 7, die beiden Rohre 11, 16, die dazwischen liegende Isolierschicht 25, die Fassung 14, die Metallisierung 22 sowie die Feder 27. Ferner kann das Heizsystem 8 noch die schematisch in Fig. 1 eingezeichnete Strom- bzw. Spannungsquelle 40 aufweisen, die an die Anschlüsse 6, 7 angeschlossen werden kann, wie durch die gestrichelten Linien 41, 42 angedeutet ist.

Beim bestimmungsgemäßen Gebrauch des erfindungsgemäßen Endoskops 1 wird ein zu betrachtendes Objekt (nicht gezeigt) über die Optik 20 sowie über im Schaft angeordnete Stablinsen 30, von denen in Fig. 4 eine eingezeichnet ist, bis zum Handgriff 5 übertragen, in dem eine Okularoptik 31 angeordnet sein kann, so daß ein Benutzer des Endoskops über die Einblicköffnung 10 das übertragene Bild des Objektes erfassen kann. Die Einblicköffnung kann, wie in Fig. 4 ersichtlich ist, durch ein hinteres Deckglas 32 verschlossen sein. Natürlich kann das Endoskop 1 auch so ausgebildet sein, daß am proximalen Ende des Handgriffes 5 eine Videokamera befestigt werden kann, die das übertragene Bild aufnimmt.

An die beiden elektrischen Anschlüsse 6, 7 ist dabei die Stromquelte 40 angeschlossen, so daß ein Stromfluß vom ersten elektrischen Anschluß 6 über das erste Rohr 11, die Fassung 14, den ersten Kontaktabschnitt 23, die Beschichtung 22 bis zum zweiten Kontaktabschnitt 24, die Feder 27, das zweite Rohr 16 bis zum zweiten elektrischen Anschluß 7 (oder in umgekehrter Richtung) vorliegt. Der Stromfluß wird dabei so eingestellt, daß aufgrund des Widerstandes der Beschichtung 22 ein Erwärmen des Deckglases 15 bewirkt wird, um ein unerwünschtes Beschlagen des Deckglases 15 zu verhindern. Der Stromfluß kann beispielsweise so eingestellt werden, daß das Deckglas auf circa 40° erwärmt wird. Dazu ist hier die Stromstärke 0,75 A bei einer angelegten Spannung von 1,25 V.

Natürlich kann die Strombeaufschlagung geregelt durchgeführt werden. Ferner ist es möglich, einen Temperatursensor im Bereich des Deckglases 15 vorzusehen, um eine Regelung auf eine einstellbare Solltemperatur durchzuführen.

## Patentansprüche

1. Endoskop mit
einem ersten Rohr (11), dessen distales Ende mit einem in einer Fassung (14) sitzenden Deckglas (15) verschlossen ist,
und einem elektrischen Heizsystem zum Erwärmen des Deckglases (15),
wobei das Heizsystem zwei elektrische Anschlüsse (6, 7), die mit einer Stromquelle verbindbar sind, sowie eine auf der Innenseite (19) des Deckglases (15) aufgebrachte elektrisch leitfähige Beschichtung (22) mit zwei voneinander beabstandeten Kontaktabschnitten (23, 24) aufweist, von denen jeder jeweils mit einem der Anschlüsse (6, 7) elektrisch verbunden ist,
**dadurch gekennzeichnet, daß** die Fassung (14) elektrisch leitfähig ist und ein erster (23) der beiden Kontaktabschnitte (23, 24) über die Fassung (14) mit dem zugeordneten Anschluß (6, 7) elektrisch verbunden ist, daß das erste Rohr (11) elektrisch leitfähig ist und die Fassung (14) mit dem dem ersten Kontaktabschnitt (23) zugeordneten Anschluß (7) elektrisch verbindet, und daß in dem ersten Rohr (11) ein zweites Rohr (16) eingesetzt ist, das elektrisch leitfähig ist, wobei zwischen beiden Rohren (11, 16) eine Isolierschicht (25) angeordnet ist und die elektrische Verbindung des zweiten Kontaktabschnittes (24) mit dem zugeordneten Anschluß (7) über das zweite Rohr (16) erfolgt.

2. Endoskop nach Anspruch 1, **dadurch gekennzeichnet, daß** eine Feder (27) zwischen dem zweiten Kontaktabschnitt (24) und dem zweiten Rohr (16) angeordnet ist, die eine elektrische Verbindung des zweiten Kontaktabschnittes (24) mit dem zweiten Rohr (16) bewirkt.

3. Endoskop nach einem der obigen Ansprüche, **dadurch gekennzeichnet, daß** das erste Rohr (11) hermetisch dicht verschlossen ist.

4. Endoskop nach einem der obigen Ansprüche, **dadurch gekennzeichnet, daß** die Beschichtung (22), in Draufsicht auf das Deckglas (15) gesehen, die Form eines offenen Ringes aufweist.

5. Endoskop nach einem der obigen Ansprüche, **dadurch gekennzeichnet, daß** im ersten Rohr (11) am distalen Ende eine Abbildungsoptik (20) angeordnet ist, die so ausgelegt ist, daß zur Abbildung eines vor dem Deckglas befindlichen Objektes nur Licht verwendet wird, das einen Teilbereich der Innenseite durchläuft, wobei die Beschichtung außerhalb des Teilbereiches auf der Innenseite aufgebracht ist.

6. Endoskop nach einem der obigen Ansprüche, **dadurch gekennzeichnet, daß** die Beschichtung als Metallisierung ausgebildet ist.

7. Endoskop nach einem der obigen Ansprüche, **dadurch gekennzeichnet, daß** der erste Kontaktabschnitt (23) direkt mit der Fassung (14) elektrisch verbunden ist.

## Claims

1. An endoscope, comprising
a first tube (11) whose distal end is sealed with a cover glass (15) disposed in a mount (14) and an electric heating system for heating the cover glass (15),
with the heating system comprising two electric connections (6, 7) which can be connected with a power source, and an electrically conductive coating (22) which is applied to the inside (19) of the cover glass (15) and comprises two mutually spaced contact sections (23, 24), of which one each is electrically connected with one of the connections (6, 7),
**characterized in that** the mount (14) is electrically conductive and a first (23) of the two contact sections (23, 24) is electrically connected via the mount (14) with the associated connection (6, 7),
that the first tube (11) is electrically conductive and electrically connects the mount (14) with the connection (7) associated with the first contact section (23),
and that a second tube (16) which is electrically conductive is inserted in the first tube (11), with an insulating layer (25) being arranged between the two tubes (11, 16) and the electric connection of the second contact section (24) with the associated connection (7) occurring via the second tube (16).

2. An endoscope according to claim 1, **characterized in that** a spring (27) is arranged between the second contact section (24) and the second tube (16) which causes an electric connection of the second contact section (24) with the second tube (16).

3. An endoscope according to one of the preceding claims, **characterized in that** the first tube (11) is sealed in a hermetically tight manner.

4. An endoscope according to one of the preceding claims, **characterized in that** the coating (22) has the form of an open ring, when seen in a top view of the cover glass (15).

5. An endoscope according to one of the preceding claims, **characterized in that** imaging optics (20) are arranged in the first tube (11) at the distal end, which imaging optics are arranged in such a way that for imaging an object disposed in front of the cover glass only light is used which passes through a predetermined partial area of the inside, with the coating outside of the partial area being applied on the inside.

6. An endoscope according to one of the preceding claims, **characterized in that** the coating is arranged as a metallization.

7. An endoscope according to one of the preceding claims, **characterized in that** the first contact section (23) is electrically directly connected with the mount (14).

## Revendications

1. Endoscope, comportant
un premier tube (11), dont l'extrémité distale est fermée par un verre de protection (15) monté dans une monture (14),
et un système de chauffage électrique pour chauffer le verre de protection (15),
ledit système de chauffage comportant deux raccords (6, 7) électriques pouvant être reliés à une source de courant, ainsi qu'un revêtement (22) électroconducteur, qui est appliqué sur la face intérieure (19) du verre de protection (15) et est muni de deux tronçons de contact (23, 24) écartés l'un de l'autre, dont chacun est relié électriquement à un des raccords (6, 7),
**caractérisé en ce que** la monture (14) est électroconductrice, et un premier (23) des deux tronçons de contact (23, 24) est relié électriquement, par l'intermédiaire de la monture (14), au raccord (6, 7) associé, **en ce que** le premier tube (11) est électroconducteur et la monture (14) est reliée électriquement au raccord (7) associé au premier tronçon de contact (23), et **en ce que** dans le premier tube (11) est enfiché un deuxième tube (16) qui est électroconducteur, une couche isolante (25) étant disposée entre les deux tubes (11, 16) et la liaison électrique du deuxième tronçon de contact (24) avec le raccord (7) associé étant assurée par l'intermédiaire du deuxième tube (16).

2. Endoscope selon la revendication 1, **caractérisé en ce qu'**un ressort (27) est agencé entre le deuxième tronçon de contact (24) et le deuxième tube (16) et génère une liaison électrique entre le deuxième tronçon de contact (24) et le deuxième tube (16).

3. Endoscope selon l'une quelconque des revendications précédentes, **caractérisé en ce que** le premier tube (11) est fermé de manière hermétiquement étanche.

4. Endoscope selon l'une quelconque des revendications précédentes, **caractérisé en ce que** le revêtement (22), par référence à une vue en élévation du verre de protection (15), possède la forme d'un anneau ouvert.

5. Endoscope selon l'une quelconque des revendications précédentes, **caractérisé en ce que** dans le premier tube (11), au niveau de l'extrémité distale, est agencée une optique de reproduction (20), qui est configurée de telle sorte que seule la lumière parcourant une portion de la face intérieure est utilisée pour reproduire un objet situé devant le verre de protection, le revêtement étant déposé sur la face intérieure en dehors de ladite portion.

6. Endoscope selon l'une quelconque des revendications précédentes, **caractérisé en ce que** le revêtement est réalisé sous la forme d'une métallisation.

7. Endoscope selon l'une quelconque des revendications précédentes, **caractérisé en ce que** le premier tronçon de contact (23) est en liaison électrique directe avec la monture (14).
